(19) **Europäisches Patentamt / European Patent Office / Office européen des brevets**

(11) **EP 4 368 032 A1**

(12) # EUROPEAN PATENT APPLICATION
published in accordance with Art. 153(4) EPC

(43) Date of publication:
**15.05.2024 Bulletin 2024/20**

(21) Application number: **22837730.5**

(22) Date of filing: **07.07.2022**

(51) International Patent Classification (IPC):
*A23L 33/17* (2016.01)    *A23L 33/115* (2016.01)
*A23L 33/125* (2016.01)    *A61K 31/198* (2006.01)
*A61K 31/20* (2006.01)    *A61K 31/23* (2006.01)
*A61K 36/48* (2006.01)    *A61K 38/02* (2006.01)
*A61K 38/39* (2006.01)    *A61P 3/02* (2006.01)
*A61P 3/12* (2006.01)

(52) Cooperative Patent Classification (CPC):
**A23L 33/115; A23L 33/125; A23L 33/17;
A61K 31/198; A61K 31/20; A61K 31/23;
A61K 36/48; A61K 38/02; A61K 38/39; A61P 3/02;
A61P 3/12**

(86) International application number:
**PCT/JP2022/026920**

(87) International publication number:
**WO 2023/282316 (12.01.2023 Gazette 2023/02)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.07.2021 JP 2021113534**

(71) Applicant: **Otsuka Pharmaceutical Factory, Inc.
Naruto-shi, Tokushima 772-8601 (JP)**

(72) Inventors:
• **YAMAOKA, Ippei
  Naruto-shi, Tokushima 772-8601 (JP)**
• **AKIYAMA, Ryosuke
  Naruto-shi, Tokushima 772-8601 (JP)**

(74) Representative: **Hoffmann Eitle
Patent- und Rechtsanwälte PartmbB
Arabellastraße 30
81925 München (DE)**

(54) **ENTERAL NUTRIENT PREPARATION**

(57)    An object of the present invention is to provide a new method for providing nutritional supplementation to a subject in poor nutrition state while preventing or suppressing refeeding syndrome, and an enteral nutrient preparation that can be used in the method. As a means for solving the problem, provided is the enteral nutrient preparation including protein and a lipid, and an amino acid as necessary, in which 90 wt% or more of the protein is not milk-derived protein, and (a) an amino acid score of a mixture of the protein and the amino acid is 100 and a lipid calorie ratio is 40% or more, or (b) an amino acid score of a mixture of the protein and the amino acid is less than 100 and a lipid calorie ratio is 15% or more.

Fig. 2

Plasma inorganic phosphorus concentrations (mg/dL)

**Description**

Technical Field

The present invention relates to enteral nutrient preparations and uses thereof.

Background Art

Refeeding syndrome is a generic term for a series of metabolic complications developed by actively providing nutritional supplementation to a subject who continues to be in a chronic malnutrition state (NPL 1 and NPL 2). That is, the refeeding syndrome is considered to be developed when a subject in poor nutrition state suddenly starts nutrition therapy. In particular, the subject in poor nutrition state who is transported to a medical institution in an emergency is likely to develop refeeding syndrome, and has often difficulty providing nutrition.

Specific symptoms of the refeeding syndrome include, for example, hypophosphatemia, hypokalemia, hypomagnesemia, vitamin $B_1$ deficiency, and the like.

Citation List

Non-patent Literature

NPL 1: Hisham Mehanna et al., 3 others, "Refeeding syndrome - awareness, prevention and management", 2009, Head & Neck Oncology, 1: 4

NPL 2: Stanga Z et al., 6 others, "Nutrition in clinical practice - the refeeding syndrome: illustrative cases and guidelines for prevention and treatment.", 2008, Eur J Clin Nutr., 62 (6): 687-94

Summary of Invention

Technical Problem

The present inventors have focused on the fact that means for providing nutritional supplementation to a subject in poor nutrition state while preventing or suppressing the refeeding syndrome is currently limited. An object of the present invention is to provide a new method for providing such nutritional supplementation, and an enteral nutrient preparation that can be used in the method.

Solution to Problem

The present inventors have extensively conducted studies for achieving the above-mentioned object, and resultantly found that when (a) an enteral nutrient preparation which does not contain milk-derived protein, has amino acid score of 100 in a mixture of protein and amino acid, and has a lipid calorie ratio of 40% or more, or (b) an enteral nutrient preparation which does not contain milk-derived protein, has amino acid score of less than 100 in a mixture of protein and amino acid, and has a lipid calorie ratio of 15% or more is used, in a subject in poor nutrition state, nutritional supplementation can be provided while refeeding syndrome is prevented or suppressed. The present invention has been completed by further conducting studies based on such findings, and includes the following aspects.

Section 1

An enteral nutrient preparation including protein and a lipid, and an amino acid as necessary, in which 90 wt% or more of the protein is not milk-derived protein, and

(a) an amino acid score of a mixture of the protein and the amino acid is 100, and a lipid calorie ratio is 40% or more, or
(b) an amino acid score of a mixture of the protein and the amino acid is less than 100, and a lipid calorie ratio is 15% or more.

Section 2

The enteral nutrient preparation according to Section 1, in which 90 wt% or more of protein source of the protein is plant-derived protein and/or collagen.

Section 3

The enteral nutrient preparation according to Section 1 or 2, including more than 3.5 g of the protein and/or the amino acid per 100 kcal in total.

Section 4

The enteral nutrient preparation according to any one of Sections 1 to 3, further including glucide.

Section 5

The enteral nutrient preparation according to any one of Sections 1 to 4, which is used for preventing or suppressing refeeding syndrome.

Section 6

The enteral nutrient preparation according to any one of Sections 1 to 5, which is used for preventing or suppressing hypophosphatemia.

Section 7

The enteral nutrient preparation according to any one of Sections 1 to 6, which is used for administration to a subject in poor nutrition state.

Section 8

A method for providing nutritional supplementation to a subject in poor nutrition state using an enteral nutrient preparation, the enteral nutrient preparation including protein and a lipid, and an amino acid as necessary, in which 90 wt% or more of the protein is not milk-derived protein, and

(a) an amino acid score of a mixture of the protein and the amino acid is 100, and a lipid calorie ratio is 40% or more, or
(b) an amino acid score of a mixture of the protein and the amino acid is less than 100, and a lipid calorie ratio is 15% or more.

Advantageous Effects of Invention

According to the present invention, it is possible to provide nutritional supplementation to a subject in poor nutrition state while preventing or suppressing symptoms of refeeding syndrome.

Brief Description of Drawings

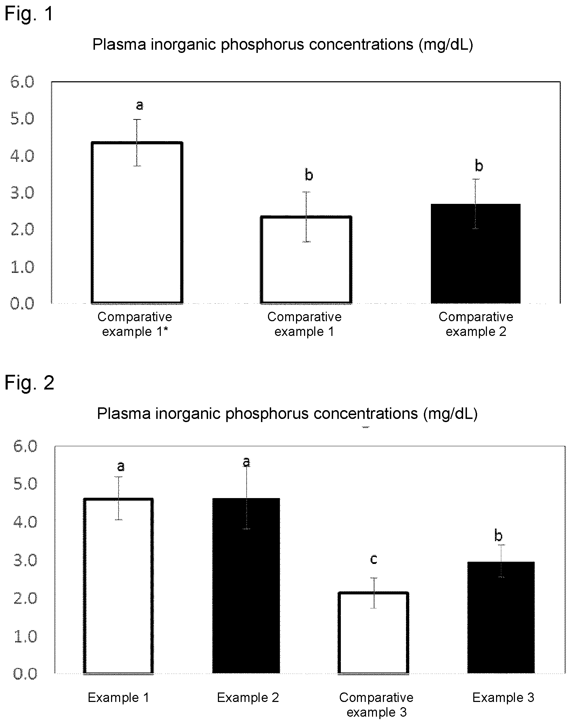

Fig. 1 is a graph showing plasma inorganic phosphorus concentrations when liquid diets of Comparative Examples 1 and 2 were administered at the time of refeeding. Data are shown as mean $\pm$ standard deviation (n = 12/group) for each group. The different sign means that there is a significant difference (p < 0.05, Fisher's Least Significant Difference Method).
Fig. 2 is a graph showing plasma inorganic phosphorus concentrations when liquid diets of Comparative Example 3 and Examples 1 to 3 were administered at the time of refeeding. Data are shown as mean $\pm$ standard deviation (n = 12/group, however, n = 11/group in Comparative Example 3 and Example 3) for each group. The different sign means that there is a significant difference (p < 0.05, multiple comparison of Holm) .

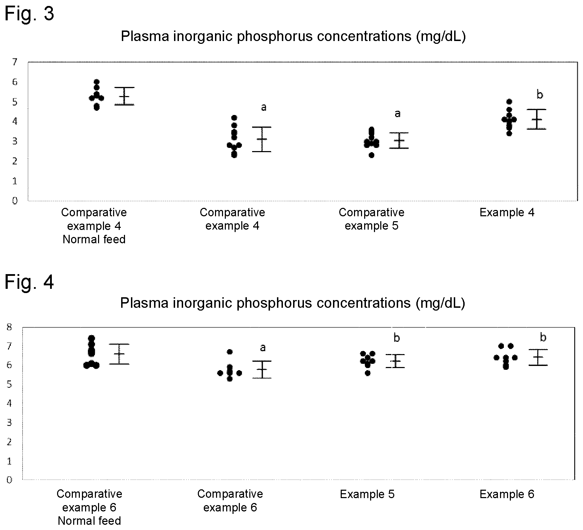

Fig. 3 is a graph showing plasma inorganic phosphorus concentrations when liquid diets of Comparative Examples 4 and 5 and Example 4 were administered at the time of refeeding. Data are shown as mean $\pm$ standard deviation (n = 10/group, however, n = 9/group in Example 4, and n = 8/group in Comparative Example 4 normal feed) for each group and the value of each individual as a dot plot. The different sign means that there is a significant difference (p < 0.05, Fisher's Least Significant Difference Method).

Fig. 4 is a graph showing plasma inorganic phosphorus concentrations when liquid diets of Comparative Example 6 and Examples 5 and 6 were administered at the time of refeeding. Data are shown as mean ± standard deviation (n = 8/group, however, n = 7/group in Comparative Example 6) for each group and the value of each individual as a dot plot. The different sign means that there is a significant difference (p < 0.05, Fisher's Least Significant Difference Method).

Description of Embodiments

1. Enteral nutrient preparation of the present invention

An enteral nutrient preparation of the present invention contains protein and a lipid, and an amino acid as necessary, and 90 wt% or more of the protein is not milk-derived protein.

Examples of the milk-derived protein include whey protein and casein. In the present invention, the term "whey protein" includes whey peptide obtained by hydrolyzing whey protein.

In the enteral nutrient preparation of the present invention, it is preferable that 95 wt% or more of the protein source is not milk-derived protein, it is more preferable that 98 wt% or more is not milk-derived protein, it is still more preferable that 99 wt% or more is not milk-derived protein, and it is most preferable that 99.9 wt% or more is not milk-derived protein. In the enteral nutrient preparation of the present invention, it is preferable that 90 wt% or more of the protein source is plant-derived protein and/or collagen. In the composition of the present invention, more preferably 95 wt% or more of the protein source of the protein, still more preferably 98 wt% or more of the protein source, still more preferably 99 wt% or more of the protein source, and most preferably 99.9 wt% or more of the protein source is plant-derived protein and/or collagen.

In the above description, examples of the origin of the plant-derived protein include soybean, wheat, pea, and rice, and protein derived from soybean is preferable. In the present invention, the "plant-derived protein" includes plant-derived peptide obtained by hydrolyzing a plant-derived protein.

In the present invention, "collagen" includes collagen peptide obtained by hydrolyzing collagen.

The enteral nutrient preparation of the present invention may contain an amino acid as necessary in addition to protein.

The above-mentioned "may contain an amino acid" means that the amino acid is added to the protein. In addition, when the term "protein source" is used in the present specification, the protein source includes the amino acid derived from the protein but does not include added amino acids.

The enteral nutrient preparation of the present invention contains the protein and/or the amino acid in an amount of preferably more than 3.5 g, more preferably 4.0 g or more, and still more preferably 5.0 g or more per 100 kcal in total in order to sufficiently provide nutritional supplementation. In addition, the total amount of the protein and/or the amino acid is preferably 8.0 g or less, more preferably 7.5 g or less, and still more preferably 7.0 g or less. The total amount of the protein and/or the amino acid per 100 kcal is preferably any of more than 3.5 g and 8.0 g or less, more than 3.5 g and 7.5 g or less, more than 3.5 g and 7.0 g or less, 4.0 to 8.0 g, 4.0 to 7.5 g, 4.0 to 7.0 g, 5.0 to 8.0 g, 5.0 to 7.5 g, or 5.0 to 7.0 g.

The range of the calorie ratio of the protein and/or the amino acid in the enteral nutrient preparation of the present invention is preferably any of more than 14 and 32% or less, more than 14 and 30% or less, more than 14 and 28% or less, 16 to 32%, 16 to 30%, 16 to 28%, 20 to 32%, 20 to 30%, and 20 to 28%.

The enteral nutrient preparation of the present invention contains a lipid in addition to the protein.

From the viewpoint of preventing or suppressing refeeding syndrome, examples of a lipid calorie ratio and an amino acid score of a mixture of protein and amino acid in the enteral nutrient preparation of the present invention include the following (a) or (b).

   (a) The amino acid score of the mixture of the protein and the amino acid is 100, and the lipid calorie ratio is 40% or more.
   (b) The amino acid score of the mixture of the protein and the amino acid is less than 100, and the lipid calorie ratio is 15% or more.

In the present specification, the "amino acid score" is calculated as follows, using the rating pattern (Table 1) (Standard Tables of Food Composition in Japan 2015 (seventh edition)) regarding the essential amino acids of a 3-10 year old child proposed by FAO/WHO/UNU in 1985 and revised in 2007.

[Table 1]

| Essential amino acids | Grading pattern (mg/g protein) |
|---|---|
| Lysine | 48 |
| Histidine | 16 |
| Total aromatic amino acids | 41 |
| Leucine | 61 |
| Isoleucine | 31 |
| Total sulfur-containing amino acids | 24 |
| Valine | 4 |
| Threonine | 25 |
| Tryptophan | 6.6 |

(1) For each of the essential amino acids listed in Table 1, the amount of the essential amino acid (mg/g protein) in the protein in the enteral nutrient preparation is determined.
(2) The amount of the essential amino acids in the protein in the enteral nutrient preparation, which is obtained in (1), is divided by the amino acid score pattern shown in Table 1 and then multiplied by 100.
(3) The minimum value is defined as the amino acid score. The amino acid score when the minimum value exceeds 100 is represented as 100. The amino acid exhibiting the minimum value is referred to as a "first limiting amino acid".

Amino acid score = (first limiting amino acid content (mg/g) in protein in enteral nutrient preparation) / (corresponding amino acid amount (mg/g) in amino acid score pattern) $\times$ 100

In the enteral nutrient preparation of the present invention, the calorie ratio of lipid, protein, and glucide can be determined by the following formula.

Lipid (%) = Lipid (g) $\times$ 9 (kcal/g)/Total energy of enteral nutrient (kcal) $\times$ 100

Protein (%) = Protein (g) $\times$ 4 (kcal/g)/Total energy of enteral nutrient (kcal) $\times$ 100

$$\text{Glucide (\%) = 100 - Protein (\%) - Lipid (\%)}$$

In the enteral nutrient preparation of the present invention, when the lipid calorie ratio and the amino acid score of the mixture of protein and amino acid are above (a), the lipid calorie ratio is preferably 40% or more, more preferably 45% or more, and most preferably 48% or more. The calorie ratio is preferably 70% or less, more preferably 65 or less, most preferably 60% or less. The range of the lipid calorie ratio is preferably any of 40 to 70%, 45 to 70%, 48 to 70%, 40 to 65%, 45 to 65%, 48 to 65%, 40 to 60%, 45 to 60%, and 48 to 60%.
In the enteral nutrient preparation of the present invention, when the lipid calorie ratio and the amino acid score of the mixture of protein and amino acid are above (b), the lipid calorie ratio is preferably 15% or more, more preferably 20% or more, and most preferably 25% or more. The calorie ratio is preferably 70% or less, more preferably 65 or less, most preferably 60% or less. The range of the lipid calorie ratio is preferably any of 15 to 70%, 20 to 70%, 25 to 70%, 15 to 65%, 20 to 65%, 25 to 65%, 15 to 60%, 20 to 60%, and 25 to 60%.
In the enteral nutrient preparation of the present invention, when the lipid calorie ratio and the amino acid score of the mixture of protein and amino acid are above (b), the amino acid score is preferably less than 100, more preferably 98 or less, and most preferably 95 or less. The amino acid score is preferably 1 or more. The range of the amino acid score is preferably any of 1 to less than 100, 1 to 98, and 1 to 95.

The type of lipid in the enteral nutrient preparation of the present invention is not particularly limited, and examples thereof include vegetable oils such as rice oil, coconut oil, soybean oil, corn oil, rapeseed oil, palm oil, safflower oil, sunflower oil, soybean oil, olive oil, cottonseed oil, peanut oil, and cacao butter; animal oils such as fish oil, beef tallow, and lard; fatty acid, medium-chain fatty acid (about 6 to 12 carbon atoms) triglyceride, docosahexaenoic acid, eicosapentaenoic acid, and the like. These lipids may be used singly or in combination of two or more kinds thereof.

When the enteral nutrient preparation of the present invention is made into a liquid form, the lipid is preferably contained in an emulsified state. When the lipid is contained in the enteral nutrient preparation of the present invention to form the emulsified state, the emulsified form is not particularly limited, and may be either an oil-in-water type or a water-in-oil type, and the oil-in-water type is preferable. When the enteral nutrient preparation of the present invention is provided in a solid form, it is desirable to use a solid oil-and-fat as the oil-and-fat.

The enteral nutrient preparation of the present invention may contain glucide.

In the present invention, the glucide is carbohydrate that is decomposed by digestive enzyme and used as an energy source. The kind of the glucide used in the present invention is not particularly limited, and examples thereof include: monosaccharides such as glucose, galactose, fructose, and xylose; disaccharides such as sucrose, lactose, and maltose; oligosaccharides such as galacto-oligosaccharide, xylo-oligosaccharide, soybean oligosaccharide, fructo-oligosaccharide, and lactosucrose; polysaccharides such as dextrin, maltodextrin, starch, and the like. These glucides may be used singly or in combination of two or more kinds thereof.

The amount of glucide in the enteral nutrient preparation of the present invention can be, for example, 4 to 20 g/100 kcal, and is preferably 6 to 15 g/100 kcal.

The enteral nutrient preparation of the present invention may contain the following components in appropriate amount as necessary.

Dietary fiber

The type of the dietary fiber is not particularly limited, and examples thereof include pectin, alginic acid, salt of alginic acid (sodium salt or the like), gellan gum, carrageenan, ghatti gum, gum arabic, karaya gum, tragacanth gum, locust bean gum, resistant starch, konjac mannan, glucomannan, $\beta$-glucan, indigestible dextrin, polydextrose, inulin, indigestible oligosaccharide, agarose, fucoidan, porphyran, laminaran, guar gum, and the like.

The amount of dietary fiber in the enteral nutrient preparation of the present invention can be, for example, 0.5 to 2.0 g/100 kcal, and is preferably 0.6 to 1.6 g/100 kcal.

Vitamin

The enteral nutrient preparation of the present invention may contain water-soluble vitamin and/or fat-soluble vitamin as vitamin.

Examples of the water-soluble vitamin contained in the enteral nutrient preparation of the present invention include vitamin B group and vitamin C. Examples of the vitamin B group include vitamin $B_1$ (thiamine), vitamin $B_2$ (riboflavin), vitamin $B_3$ (niacin), vitamin $B_5$ (pantothenic acid), vitamin $B_6$, vitamin $B_7$ (biotin), vitamin $B_9$ (folic acid), vitamin $B_{12}$ (cyanocobalamin), and the like. Examples of the fat-soluble vitamin include vitamin A, vitamin D (particularly cholecalciferol), vitamin E, vitamin K, and the like.

The amount of vitamin C in the enteral nutrient preparation of the present invention can be, for example, 20 to 160 mg/100 kcal, and is preferably 30 to 140 mg/100 kcal, more preferably 40 to 120 mg/100 kcal.

As vitamin $B_1$, thiamine, thiamine hydrochloride, and the like can be used. The amount of vitamin $B_1$ in the enteral nutrient preparation of the present invention can be, for example, 0.1 to 10.0 mg/100 kcal, and is preferably 0.2 to 7.0 mg/100 kcal as thiamine.

As vitamin $B_2$, riboflavin, sodium riboflavine phosphate, flavin mononucleotide, and the like can be used. The amount of vitamin $B_2$ in the enteral nutrient preparation of the present invention can be, for example, 0.1 to 0.6 mg/100 kcal, and is preferably 0.2 to 0.5 mg/100 kcal as riboflavin.

As vitamin $B_6$, salts of pyridoxine such as pyridoxine and pyridoxine hydrochloride, and the like can be used. The amount of vitamin $B_6$ in the enteral nutrient preparation of the present invention can be, for example, 0.1 to 1.0 mg/100 kcal, and is preferably 0.2 to 0.8 mg/100 kcal as pyridoxine.

The amount of folic acid in the enteral nutrient preparation of the present invention can be, for example, 10 to 120 pg/100 kcal, and is preferably 20 to 100 pg/100 kcal.

As vitamin $B_{12}$, cyanocobalamin, hydroxocobalamin acetate, methylcobalamin, and the like can be used. The amount of vitamin $B_{12}$ in the enteral nutrient preparation of the present invention can be, for example, 0.1 to 1.2 pg/100 kcal, and is preferably 0.2 to 1.0 pg/100 kcal.

As the niacin, for example, nicotinic acid amide can be preferably used. The amount of niacin in the enteral nutrient preparation of the present invention can be, for example, 1.0 to 12 mgNE/100 kcal, and is preferably 2.0 to 10 mgNE/100 kcal.

As the pantothenic acid, panthenol can be preferably used. The amount of panthenol in the enteral nutrient preparation of the present invention can be, for example, 0.1 to 8.0 mg/100 kcal, and is preferably 0.5 to 4.0 mg/100 kcal.

The amount of biotin in the enteral nutrient preparation of the present invention can be, for example, 1.0 to 15 pg/100 kcal, and is preferably 2.0 to 12 pg/100 kcal.

As vitamin A, retinol palmitate can be preferably used. A vitamin A oil obtained by dissolving the retinol palmitate in oil can also be used. The amount of vitamin A in the enteral nutrient preparation of the present invention can be, for example, 20 to 200 $\mu$g RE/100 kcal, and is preferably 40 to 120 $\mu$g RE/100 kcal.

As vitamin D, cholecalciferol (vitamin $D_3$) can be preferably used. The amount of vitamin D in the enteral nutrient preparation of the present invention can be, for example, 0.1 to 8.0 pg/100 kcal, and is preferably 0.5 to 4.0 pg/100 kcal.

As vitamin E, tocopherol acetate can be preferably used. The amount of vitamin E in the enteral nutrient preparation of the present invention can be, for example, 1.0 to 12 mg/100 kcal, and is preferably 2.0 to 10 mg/100 kcal.

As vitamin K, phytonadione (vitamin $K_1$) can be preferably used. The amount of vitamin K in the enteral nutrient preparation of the present invention can be, for example, 1.0 to 30 pg/100 kcal, and is preferably 5.0 to 20 pg/100 kcal.

Mineral (ash content)

The enteral nutrient preparation of the present invention may contain mineral. As the mineral, those used for normal enteral nutrient preparations can be appropriately selected. Specific examples thereof include sodium, chlorine, potassium, calcium, magnesium, phosphorus, iron, zinc, copper, manganese, iodine, selenium, chromium, molybdenum, and the like. In the enteral nutrient preparation of the present invention, two or more of these can be blended.

The amount of sodium in the enteral nutrient preparation of the present invention can be, for example, 10 to 600 mg/100 kcal, and is preferably 50 to 400 mg.

The amount of chlorine in the enteral nutrient preparation of the present invention can be, for example, 10 to 600 mg/100 kcal, and is preferably 50 to 400 mg.

The amount of potassium in the enteral nutrient preparation of the present invention can be, for example, 10 to 600 mg/100 kcal, and is preferably 50 to 400 mg.

The amount of calcium in the enteral nutrient preparation of the present invention can be, for example, 10 to 400 mg/100 kcal, and is preferably 20 to 200 mg.

The amount of magnesium in the enteral nutrient preparation of the present invention can be, for example, 5.0 to 200 mg/100 kcal, and is preferably 10 to 100 mg.

The amount of phosphorus in the enteral nutrient preparation of the present invention can be, for example, 10 to 400 mg/100 kcal, and is preferably 20 to 200 mg.

The amount of iron in the enteral nutrient preparation of the present invention can be, for example, 0.1 to 4.0 mg/100 kcal, and is preferably 0.2 to 2.0 mg.

The amount of zinc in the enteral nutrient preparation of the present invention can be, for example, 0.1 to 6.0 mg/100 kcal, and is preferably 0.2 to 4.0 mg.

The amount of copper in the enteral nutrient preparation of the present invention can be, for example, 0.01 to 2.0 mg/100 kcal, and is preferably 0.02 to 1.0 mg.

The amount of manganese in the enteral nutrient preparation of the present invention can be, for example, 0.1 to 2.0 mg/100 kcal, and is preferably 0.2 to 1.0 mg.

The amount of iodine in the enteral nutrient preparation of the present invention can be, for example, 1.0 to 60 pg/100 kcal, and is preferably 5.0 to 40 $\mu$g.

The amount of selenium in the enteral nutrient preparation of the present invention can be, for example, 0.5 to 20 pg/100 kcal, and is preferably 1.0 to 10 $\mu$g.

The amount of chromium in the enteral nutrient preparation of the present invention can be, for example, 0.5 to 20 pg/100 kcal, and is preferably 1.0 to 10 $\mu$g.

The amount of molybdenum in the enteral nutrient preparation of the present invention can be, for example, 0.5 to 20 pg/100 kcal, and is preferably 1.0 to 10 $\mu$g.

Other components and dosage forms

The amount of water in the enteral nutrient preparation of the present invention can be, for example, 30 to 150 g/100 kcal, and is preferably 40 to 120 g/100 kcal.

The enteral nutrient preparation of the present invention can have a calorie concentration of, for example, 60 to 170 kcal/100 mL, and the calorie concentration is preferably 70 to 150 kcal/100 mL, more preferably 80 to 130 kcal/100 mL, and most preferably 90 to 120 kcal/100 mL.

The enteral nutrient preparation of the present invention may be provided in a liquid form, or may be provided in a solid form such as a powder form or a granular form, but is preferably provided as liquid diets in the liquid form in terms of easy intake.

## 2. Method for producing the enteral nutrient preparation of the present invention

The enteral nutrient preparation of the present invention is prepared by a production method according to its form. For example, when the enteral nutrient preparation of the present invention is in a liquid form, the enteral nutrient preparation can be produced by adding a predetermined amount of the above-described lipid, carbohydrate, protein, and other components blended as necessary to water and mixing them. When the enteral nutrient preparation of the present invention is brought into an emulsified state, water may be added to the mixture and emulsification may be performed with a homogenizer.

When the enteral nutrient preparation of the present invention is provided in the liquid form, it is desirable to subject the enteral nutrient preparation to heat sterilization treatment before or after filling the enteral nutrient preparation in a container such as an aluminum pouch or a soft bag. When subjected to the heat sterilization treatment as described above, the preservation stability can be enhanced. The temperature condition of the heat sterilization treatment is not particularly limited, and is, for example, 110 to 150 °C, preferably 120 to 145 °C. Specific examples of the heat sterilization treatment include pressure heat sterilization, ultrahigh temperature instantaneous sterilization (UHT), and high temperature short time sterilization (HTST).

When the enteral nutrient preparation of the present invention is provided in a solid form, the enteral nutrient preparation can be produced by adding and mixing predetermined amounts of the above-described lipid, carbohydrate, protein, and other components blended as necessary. In addition, after a predetermined amount of each of the above components is added to and mixed with water, the mixture may be prepared in a powder form or a granular form by spray drying, freeze drying, fluidized bed drying, vacuum drying under reduced pressure, or the like, or may be prepared in a granular form by being subjected to a fluid-bed granulation method, a fluid-bed multifunctional granulation method, a spray drying granulation method, a rolling granulation method, a stirring granulation method, or the like.

## 3. Use of the enteral nutrient preparation of the present invention

The enteral nutrient preparation of the present invention:

(a) does not contain milk-derived protein, has an amino acid score of 100 in a mixture of protein and amino acid, and has a lipid calorie ratio of 40% or more; or

(b) does not contain the milk-derived protein, has an amino acid score of less than 100 in a mixture of protein and amino acid, and has a lipid calorie ratio of 15% or more.

This makes it possible to prevent or suppress refeeding syndrome by administering the enteral nutrient preparation of the present invention to a subject in poor nutrition state.

The refeeding syndrome is a generic term for a series of metabolic complications developed by actively providing nutritional supplementation to a subject in poor nutrition state. Specific symptoms of the refeeding syndrome include, for example, hypophosphatemia, hypokalemia, hypomagnesemia, vitamin $B_1$ deficiency, and the like.

By ingestion or administration of the composition of the present invention, in particular, hypophosphatemia can be prevented or suppressed.

The enteral nutrient preparation of the present invention is preferably used for a subject in poor nutrition state who may develop the refeeding syndrome.

Examples of such subjects include subjects to which one or more of the following criteria (a) to (d) defined in the NICE guidelines apply:

(a) BMI is less than 16 kg/m$^2$
(b) 15% or greater unintentional weight loss in the last 3 to 6 months
(c) Fasting for 10 days or more
(d) Hypokalemia, hypophosphatemia, hypomagnesemia before refeeding,

or subjects who meet two or more of the following criteria (e) to (h) :

(e) BMI of less than 18.5 kg/m$^2$
(f) 10% or greater unintentional weight loss in the last 3 to 6 months
(g) Fasting for 5 or more days

(h) Has a history of alcohol dependence or a history of use of insulin, chemotherapeutic antacids, or diuretics.

In addition, a poor nutrition subject can be classified into a marasmus type in which both protein and calorie are insufficient, a kwashiorkor type in which protein shortage is more serious than calorie shortage, and a marasmus-kwashiorkor type which is an intermediate type of both. The enteral nutrient preparation of the present invention can effectively prevent or suppress the refeeding syndrome by being administered to the marasmus-kwashiorkor type undernutrition subject and the kwashiorkor type undernutrition subject, in particular.

In the enteral nutrient preparation of the present invention, for example, preferably 100 to 2000 kcal, more preferably 200 to 1600 kcal can be administered per day. In particular, in order to prevent or suppress the refeeding syndrome, it is preferable to start at 10 kcal/kg/day and gradually increase the amount over 4 to 7 days. In addition, in the case of a subject with extreme undernutrition having a BMI of less than 14 kg/m$^2$, it is preferable to start at 5 kcal/kg/day. As a result, it is possible to provide nutritional supplementation to a subject in poor nutrition state while preventing or suppressing the refeeding syndrome. As described above, it is possible to prevent or suppress the refeeding syndrome by using the enteral nutrient preparation of the present invention.

The method for ingesting or administering the enteral nutrient preparation of the present invention may be any method as long as it is ingested or administered in a liquid form. For example, in the case of use for a person without swallowing disorders, oral ingestion is applicable, and in the case of use for a person with swallowing disorders, administration via a gastrostomy tube (catheter), a nasogastric tube (catheter), or the like is applicable.

Examples

Hereinafter, the present invention will be described with reference to Examples, but the present invention is not limited to these Examples and the like.

<Test 1> Relationship between the enteral nutrient preparations blended with proteins having different lipid calorie ratios and amino acid scores and hypophosphatemia

A group in which Wistar male rats (7- week-old at the time of purchase, Charles River Laboratories Japan, Inc.) was acclimatized and then ingested purified feed containing 2.5% of a protein (Hereinafter, the feed is referred to as "low protein feed".) for 3 weeks or more and a group in which purified feed containing 20% of a protein (Hereinafter, the feed is referred to as "normal feed".) was ingested for 3 weeks or more were prepared. Each rat had free access to tap water. The composition of each feed is shown in Table 2.

[Table 2]

| Raw materials | Unit | Normal feed | Low protein feed |
|---|---|---|---|
| Casein | | 20 | 2.5 |
| L-methionine | | 0.32 | 0.04 |
| βcorn starch | | 33.45 | 34.15 |
| αcorn starch | % (w/w) | 10 | 22 |
| Sucrose | | 21.73 | 26.81 |
| Corn oil | | 5 | 5 |
| Cellulose powder p | | 5 | 5 |
| AIN-93G mineral mix | | 3.5 | 3.5 |
| AIN-93 vitamin mix | | 1 | 1 |
| Total | | 100 | 100 |

Each rat was provided with a gastrostomy as a route for administering the enteral nutrient preparation. After an overnight fast from 5:00 PM the day before the surgery, an abdominal median incision was made under isoflurane anesthesia. The stomach was exposed, a fistula for inserting a feeding tube was made at one place on the pylorus side, and the feeding tube was inserted and then fixed with sutures. The other end of the feeding tube was led out to the back via a subcutaneous route and connected to a syringe for administration solution via a harness.

Each rat with a gastrostomy was housed in a metabolic cage. After the surgery, an antibiotic (ampicillin) was injected intramuscularly at a dose of 5 mg/100 μL/rat.

Each rat was refed using the enteral nutrition preparations (Example 1 to 3, Comparative Example 1 to 3) described in Table 3.

[Table 3]

| | Protein | | | Lipid | Glucide |
|---|---|---|---|---|---|
| | Type | Amino acid | Calories | Calories | Calories |
| | | Score | Ratio (%) | Ratio (%) | Ratio (%) |
| Comparative example 1 | Whey peptide | 100 | 24 | 25 | 51 |
| Comparative example 2 | Whey peptide | 100 | 24 | 50 | 26 |
| Comparative example 3 | Collagen + amino acid | 100 | 24 | 25 | 51 |
| Example 1 | Collagen | 1 | 24 | 25 | 51 |
| Example 2 | Collagen | 1 | 24 | 50 | 26 |
| Example 3 | Collagen + amino acid | 100 | 24 | 50 | 26 |

The enteral nutrient preparations shown in Table 3 were prepared so that the amount of calorie was 100 kcal/100 mL.

In the enteral nutrient preparations of Comparative Examples 1 and 2, whey peptide (manufactured by Arla Foods Ingredients) was blended as protein, and in the enteral nutrient preparations of Comparative Example 3 and Example 1 to 3, collagen peptide (manufactured by Nippi, Incorporated) was blended. In the enteral nutrient preparations of Examples 1 and 2, only collagen peptide was used as the protein source, and in the enteral nutrient preparations of Comparative Example 3 and Example 3, amino acid was added so that the amino acid score was 100, with reference to the score pattern for ages 3 to 10 years defined by the International Organizations (FAO/WHO/UNU) in 2007 (Table 10). In each enteral nutrient preparation, protein and amino acid were blended so as to be 6 g/100 kcal (calorie ratio 24%), respectively.

In each enteral nutrient preparation, vegetable oil and medium-chain fatty acid triglyceride were blended as lipid at a ratio of 1: 1. In addition, maltodextrin was used as glucide.

In Comparative Examples 1 and 3 and Example 1, the calorie ratio of lipid and glucide in each enteral nutrient preparation was 25% (2.8 g/100 kcal) and 510 (12.8 g/100 kcal), respectively, and in Comparative Example 2 and Examples 2 and 3, the calorie ratio was 50% (2.8 g/100 kcal) and 26% (6.5 g/100 kcal), respectively.

The minerals and vitamins shown in Table 4 were blended in the enteral nutrient preparation of Comparative Example 1. The enteral nutrient preparations of Comparative Examples 2 and 3 and Example 1 to 3 also contained the minerals and vitamins shown in Table 4. Phosphorus and calcium were adjusted so that the concentration of phosphorus and calcium in the enteral nutrient preparation were the same as in Comparative Example 1, including the concentration of phosphorus and calcium derived from protein and the like.

[Table 4]

| Components | | Units | Contents (/100 kcal)) |
|---|---|---|---|
| Minerals, etc. | Sodium | mg | 61.4 |
| | Potassium | mg | 188 |
| | Magnesium | mg | 26.9 |
| | Calcium | mg | 112 |
| | Phosphorus | mg | 57.3 |
| | Chromium | mg | 2.57 |
| | Molybdenum | mg | 4.75 |
| | Manganese | mg | 0.336 |
| | Iron | mg | 0.633 |
| | Copper | mg | 0.0829 |
| | Zinc | mg | 1.45 |
| | Selenium | mg | 6.38 |
| | Iodine | mg | 13.75 |
| Vitamins | Vitamin B1 | mg | 0.225 |
| | Vitamin B2 | mg | 0.2375 |
| | Niacin | mgNE | 2.25 |
| | Vitamin B6 | mg | 0.3 |
| | Folic acid | mg | 30 |
| | Vitamin B12 | mg | 0.3 |
| | Biotin | $\mu$gRE | 4.25 |
| | Pantothenic acid | mg | 1.25 |
| | Vitamin C | mg | 52.5 |
| | Vitamin A | mg | 67.5 |
| | Vitamin E | mg | 2.375 |
| | Vitamin D | mg | 1.25 |
| | Vitamin K | mg | 6.25 |

In each enteral nutrient preparation, 0.4 g of gum ghatti and 0.067 g of cellulose preparation were blended per 100 kcal as dietary fibers.

These enteral nutrient preparations were prepared by adding the ingredients to water and mixing them with a mixer, then homogenizing them (50 MPa, 2 passes) with a high pressure homogenizer (LAB -1000, manufactured by APV Inc.), filling a pouch with the homogenized enteral nutrient preparations (oil-in-water emulsion compositions), and subjecting the pouch to heat sterilization treatment (121 °C, 10 min).

Before refeeding, each rat was blood collected from intravenous and the inorganic phosphorus concentration in the plasma was quantified by a colorimetric method developed on a Clinical Chemistry Analyzer (Fuji Dry Chem Slide IP-P, manufactured by Fujifilm Medical Co., Ltd.), and the rats were grouped so that the values did not vary. The fasting time until the start of refeeding was set to 24 hours, and during this fasting period, gastrostomy, blood collection, phosphorus concentration measurement, and grouping were performed. After the grouping, the enteral nutrient preparations described in Table 3 were continuously administered over 16 hours from the gastrostomy tube using a syringe pump so that the total amount of calories to be administered was 210 kcal per kilogram of body weight. After completion of the administration, laparotomy was performed under isoflurane anesthesia, blood was collected from the abdominal artery, plasma was fractionated, and the concentration of inorganic phosphorus in the plasma was measured by an enzymatic method (Determiner L, manufactured by Hitachi Chemical Diagnostics Systems Co., Ltd.).

The results are shown in Table 5 and Figs. 1 and 2. In the present specification, the value of the plasma inorganic phosphorus concentration indicates mean $\pm$ standard deviation. For Fig.1, data are shown as mean $\pm$ standard deviation (n = 12/group) for each group. Comparative Example 1 * shows the group in which normal feed was taken before refeeding and Comparative Example 1 was administered, and the other groups received low protein feed before refeeding and administered each Comparative Example. The different sign means that there is a significant difference ($p < 0.05$, Fisher's least significant difference method). For Fig. 2, data are shown as mean $\pm$ standard deviation (n = 12/group, however, n = 11/group in Examples 1 and 3) for each group. The different sign means that there is a significant difference ($p < 0.05$, multiple comparison of Holm) .

For (1) to (3) in Table 5, the tests were performed by Fisher's Least Significant Difference Method. The "n.s." in Table 5 (3) and "$p < 0.05$" in Table 5 (1) indicate that "there is no significant difference" and "there is a significant difference with $p < 0.05$", respectively as compared with the rat group that was taken low protein feed before refeeding and was taken the enteral nutrient preparation of Comparative Example 1 in refeeding. In addition, (4) to (7) in Table 5 were tested by multiple comparison of Holm. In Table 5, "$p < 0.05$" in (5), (6), and (7) indicates that "there is a significant difference with $p < 0.05$" as compared with the rat group that was taken low protein feed before refeeding and was taken the enteral nutrient preparation of Comparative Example 3 (n = 12 (However, n = 11 in Examples 1 and 3)).

[Table 5]

| No. | Feed administered before refeeding | Enteral nutrient preparation administered in refeeding | Plasma inorganic phosphorus concentrations after refeeding (mg/dL) |
|---|---|---|---|
| 1 | Normal feed | Comparative example 1 | 4.4 $\pm$ 0.6 (p<0.05) |
| 2 | Low protein feed | Comparative example 1 | 2.4 $\pm$ 0.7 |
| 3 | | Comparative example 2 | 2.7 $\pm$ 0.7 (n.s.) |
| 4 | | Comparative example 3 | 2.1 $\pm$ 0.4 |
| 5 | | Example 1 | 4.6 $\pm$ 0.6 (p<0.05) |
| 6 | | Example 2 | 4.6 $\pm$ 0.8 (p<0.05) |
| 7 | | Example 3 | 3.0 $\pm$ 0.4 (p<0.05) |

The enteral nutrient preparation containing milk-derived protein

As compared with the rat (Table 5 (1)) in which normal feed was taken before refeeding and the enteral nutrient preparation (Comparative Example 1) in which the whey peptide was mixed as protein source was taken in refeeding, in the rat (Table 5 (2)) in which the low protein feed was taken before refeeding and the enteral nutrient preparation (Comparative Example 1) in which the whey peptide was mixed as the protein source was taken in refeeding, the plasma inorganic phosphorus concentration was obviously decreased and developed hypophosphatemia.

In refeeding, the plasma inorganic phosphorus concentration was also decreased in the rat (Table 5 (3)) fed with the enteral nutrient preparation (Comparative Example 2) in which the whey peptide was blended in the protein source and the lipid calorie ratio was increased from 25% to 50%, and the degree of decrease was the same.

That is, regardless of the lipid calorie ratio, it was found that the hypophosphatemia was developed by administering the enteral nutrient preparation containing milk-derived protein as protein source to a subject in poor nutrition state.

The enteral nutrient preparation containing protein having amino acid score of less than 100

Also in the rat (Table 5 (4)) that had ingested the enteral nutrient preparation (Comparative Example 3) in which collagen peptide was blended as protein source, the amino acid was added thereto, and the amino acid score was 100 in refeeding, the plasma inorganic phosphorus concentration decreased, and the hypophosphatemia was developed.

On the other hand, in refeeding, the rat (Table 5 (5)) that had ingested the enteral nutrient preparation (Example 1) in which only the collagen peptide was blended as protein source surprisingly had significantly high plasma inorganic phosphorus concentration, and symptoms of the hypophosphatemia were suppressed.

Also in the rat (Table 5 (6)) fed with the enteral nutrient preparation (Example 2) in which the lipid calorie ratio was increased from 25% to 50% in refeeding, the plasma inorganic phosphorus concentration was significantly high, and the symptoms of the hypophosphatemia were suppressed.

The group having significantly high plasma inorganic phosphorus concentration has the amino acid score of less than 100. On the other hand, the group in which the plasma inorganic phosphorus concentration decreased was the group in which the amino acid was added to the collagen peptide, and the amino acid score was set to 100. The amino acid score of the whey peptide, which is protein source of the enteral nutrient preparations of Comparative Examples 1 and 2, is also 100.

That is, it was suggested that by administering the enteral nutrient preparation containing protein having the amino acid score of less than 100 as the protein source to a subject in poor nutrition state, the hypophosphatemia was prevented or suppressed regardless of the lipid calorie ratio.

The enteral nutrient preparation in which milk-derived protein is not blended, the amino acid score of protein is 100, and the calorie ratio is high

In refeeding, in the rat (Table 5 (7)) fed with the enteral nutrient preparation (Example 3) in which the protein source was blended with the collagen peptide and the amino acid and the lipid calorie ratio was increased from 25% to 50%, surprisingly, the plasma inorganic phosphorus concentration was significantly high and the symptoms of hypophosphatemia were suppressed although the amino acid score was 100.

In refeeding, in the rat (Table 5 (4)) to which the protein source was blended with the collagen peptide and the amino acid and to which the enteral nutrient preparation (Comparative Example 3, amino acid score 100) having the lipid calorie ratio of 25% was ingested, the plasma inorganic phosphorus concentration decreased. Therefore, it was suggested that even in the case of the protein source having the amino acid score of 100, when the protein was not milk-derived protein but the lipid calorie ratio was high (For example, when the content is 40% or more,), the hypophosphatemia was prevented or suppressed.

<Test 2> Relationship between the enteral nutrient preparations blended with proteins having different amino acid scores and the hypophosphatemia

As in Test 1, a group in which low protein feed was ingested for 3 weeks or more and a group in which normal feed was ingested for 3 weeks or more were prepared.

Each rat was refed using enteral nutrient preparations (Comparative Examples 4 and 5 and Example 4) described in Table 6.

[Table 6]

| | Protein | | | Lipid | Glucide |
|---|---|---|---|---|---|
| | Type | Amino acid score | Calorie ratio (%) | Calorie ratio (%) | Calorie ratio (%) |
| Comparative example 4 | Whey peptide | 100 | 24 | 38 | 38 |
| Comparative example 5 | Collagen + soybean peptide + amino acid | 100 | 24 | 38 | 38 |
| Example 4 | Soybean peptide | 93 | 24 | 38 | 38 |

The enteral nutrient preparations shown in Table 6 were prepared so that the amount of calorie was 100 kcal/100 mL.

In the enteral nutrient preparation of Comparative Example 4, whey peptide (manufactured by Arla Foods Ingredients) was blended as protein. In the enteral nutrient preparation of Comparative Example 5, collagen peptide (manufactured by Nippi, Incorporated) and soybean peptide (manufactured by FUJI OIL CO., LTD.) were blended as proteins, and the amino acid was further added so that the amino acid score was 100 (Table 10), with reference to the score pattern for ages 3 to 10 defined by the international organizations (FAO/WHO/UNU) in 2007. The enteral nutrient preparation of Example 5 contained the soybean peptide. In each enteral nutrient preparation, protein and amino acid were blended so as to be 6 g/100 kcal (calorie ratio 24%), respectively.

In each enteral nutrient preparation, vegetable oil and medium-chain fatty acid triglyceride were blended as lipid at a ratio of 1: 1. In addition, maltodextrin was used as glucide.

The calorie ratio of the lipid and the glucide of each enteral nutrient preparation was 38% (2.8 g/100 kcal) and 38% (12.8 g/100 kcal), respectively.

The blending amounts of mineral, vitamin, and dietary fiber in each enteral nutrient preparation are the same as in Test 1.

After being divided into groups in the same manner as in Test 1, the enteral nutrient preparations described in Table 6 were continuously administered over 16 hours from the gastrostomy tube using a syringe pump so that the total amount of calories to be administered was 210 kcal per kilogram of body weight. After completion of the administration, laparotomy was performed under isoflurane anesthesia, blood was collected from the abdominal artery, plasma was fractionated, and the concentration of inorganic phosphorus in the plasma was measured by an enzymatic method (Determiner L, manufactured by Hitachi Chemical Diagnostics Systems Co., Ltd.).

The results are shown in Table 7 and Fig. 3. In the present specification, the value of the plasma inorganic phosphorus concentration indicates mean ± standard deviation. In Fig. 3, the data shows the mean ± standard deviation (n = 10/group, however, n = 9 in Example 2 and n = 8 in Comparative Example 1, normal feed) for each group and the value of each individual as a dot plot. The different sign means that there is a significant difference ($p < 0.05$, Fisher's least significant difference method).

[Table 7]

| No. | Feed administered before refeeding | Enteral nutrient preparation administered in refeeding | Plasma inorganic phosphorus concentrations after refeeding (mg/dL) |
|---|---|---|---|
| 8 | Normal feed | Comparative example 4 | 5.3 ± 0.4 (p<0.05) |
| 9 | Low protein feed | Comparative example 4 | 3.1 ± 0.6 |
| 10 | | Comparative example 5 | 3.0 ± 0.8 (n.s.) |
| 11 | | Example 4 | 4.1 ± 0.5 (p<0.05) |

As compared with the rat to which the normal feed was taken before refeeding (Table 7 (8)), in the rat in poor nutrition state (Table 7 (9)) to which the low protein feed was taken before refeeding, when the enteral nutrient preparation (Comparative Example 4) containing the same whey peptide was administered in refeeding, the plasma inorganic phosphorus concentration was obviously decreased (Student's t test: $p < 0.05$), and hypophosphatemia was developed. In addition, in refeeding, the rat (Table 7 (10)) that had ingested the enteral nutrient preparation (Comparative Example 5) in which collagen peptide and soybean peptide were blended as the protein source and the amino acid was added so that the amino acid score was 100 developed hypophosphatemia with the plasma inorganic phosphorus concentration unchanged, as compared with Table 7 (9).

Test 1 suggested that even protein source having amino acid score of 100 prevents or suppresses hypophosphatemia if the protein is not milk-derived protein and the lipid calorie ratio is high (For example, when the content is 40% or more,), but it was found that hypophosphatemia is developed when the lipid calorie ratio is about 38% (Table 5 (7)).

In refeeding, the rat (Table 7 (11)) that had ingested the enteral nutrient preparation (Example 4) containing soybean peptide as the protein source and having the amino acid score of 93 had significantly high plasma inorganic phosphorus concentration, and hypophosphatemia was suppressed.

In Test 1, it was suggested that hypophosphatemia was prevented or suppressed by administering the enteral nutrient preparation containing protein having amino acid score of less than 100 as protein source to a subject in poor nutrition state, but a similar result could be confirmed also in Test 2.

For the test 2, Fisher's least significant difference method was used. "n.s." and "p < 0.05" in Table 7 indicate that "there is no significant difference" and "there is a significant difference with $p < 0.05$", respectively as compared with the rat group that was taken low-protein feed before refeeding and was refed with the enteral nutrient preparation of Comparative Example 4 (n = 10 (However, n = 8 in the group taken normal feed before refeeding, and n = 9 in the group receiving the enteral nutrient preparation of Example 4.)).

<Test 3> Relationship 2 between enteral nutrient preparations blended with proteins having different amino acid scores and hypophosphatemia

As in Test 1, a group in which low protein feed was ingested for 3 weeks or more and a group in which normal feed was ingested for 3 weeks or more were prepared.

Each rat was refed with the enteral nutrient preparation (Comparative Example 6 and Examples 5 and 6) described in Table 8.

[Table 8]

| | Protein | | | Lipid | Glucide |
|---|---|---|---|---|---|
| | Type | Amino acid score | Calorie ratio (%) | Calorie ratio (%) | Calorie ratio (%) |
| Comparative example 6 | Whey peptide | 100 | 24 | 50 | 26 |
| Example 5 | Collagen + soybean peptide + amino acid | 10 | 24 | 50 | 26 |
| Example 6 | Soybean peptide | 93 | 24 | 50 | 26 |

The enteral nutrient preparations shown in Table 8 were prepared so that the amount of calorie was 100 kcal/100 mL.

In the enteral nutrient preparation of Comparative Example 6, a whey peptide (manufactured by Arla Foods Ingredients) was blended as protein. In the enteral nutrient preparation of Example 5, collagen peptide (manufactured by Nippi, Incorporated) and soybean peptide (manufactured by FUJI OIL CO., LTD.) were blended as proteins, and an amino acid was further added so that the amino acid score was 100 (Table 10), with reference to the score pattern for ages 3 to 10 defined by the international organizations (FAO/WHO/UNU) in 2007. The enteral nutrient preparation of Example 6 contained soybean peptide. In each enteral nutrient preparation, protein and amino acid were blended so as to be 6 g/100 kcal (calorie ratio 24%), respectively.

In each enteral nutrient preparation, vegetable oil and medium-chain fatty acid triglyceride were blended as lipid at a ratio of 1: 1. In addition, maltodextrin was used as glucide.

The calorie ratio of the lipid and the glucide of each enteral nutrient preparation was 50% (5.6 g/100 kcal) and 26% (6.5 g/100 kcal), respectively.

The blending amounts of mineral, vitamin, and dietary fiber in each enteral nutrient preparation are the same as in Test 1.

After being divided into groups in the same manner as in Test 1, the enteral nutrient preparations described in Table 8 were continuously administered over 16 hours from the gastrostomy tube using a syringe pump so that the total amount of calories to be administered was 210 kcal per kilogram of body weight. After completion of the administration, laparotomy was performed under isoflurane anesthesia, blood was collected from the abdominal artery, plasma was fractionated, and the concentration of inorganic phosphorus in the plasma was measured by an enzymatic method (Determiner L, manufactured by Hitachi Chemical Diagnostics Systems Co., Ltd.).

The results are shown in Table 9 and Fig. 4. In the present specification, the value of the plasma inorganic phosphorus concentration indicates mean ± standard deviation. In Fig. 4, the data shows mean ± standard deviation (n = 8/group, however, n = 7 in Comparative Example 3) of each group and the value of each individual as a dot plot. The different sign means that there is a significant difference ($p < 0.05$, Fisher's least significant difference method).

[Table 9]

| No. | Feed administered before refeeding | Enteral nutrient preparation administered in refeeding | Plasma inorganic phosphorus concentrations after refeeding (mg/dL) |
|---|---|---|---|
| 12 | Normal feed | Comparative example 6 | 6.6 ± 0.5 (p<0.05) |
| 13 | Low protein feed | Comparative example 6 | 5.8 ± 0.4 |
| 14 | | Example 5 | 6.2 ± 0.3 (p<0.05) |
| 15 | | Example 6 | 6.6 ± 0.8 (p<0.05) |

As compared with the rat to which the normal feed was taken before refeeding (Table 9 (12)), when the enteral nutrient preparation (Comparative Example 6) containing the same whey peptide was administered in refeeding to the rat in poor nutrition state (Table 9 (13)) to which the low protein feed was taken before refeeding, the plasma inorganic phosphorus concentration was obviously decreased (Student's t test: $p < 0.05$), and the hypophosphatemia was developed.

In refeeding, the rat (Table 9 (14)) that had ingested the enteral nutrient preparation (Example 5) in which the collagen peptide and the soybean peptide were blended as protein source and the amino acid was added so that the amino acid score was 100 had significantly higher plasma inorganic phosphorus concentration and suppressed hypophosphatemia as compared with the rat (Table 9 (13)) that had ingested the enteral nutrient preparation (Comparative Example 6) in which the whey peptide was blended as protein source.

Although Test 1 suggested that even the protein source having the amino acid score of 100 prevents or suppresses the hypophosphatemia if the protein is not the milk-derived protein and the lipid calorie ratio is high (For example, when the content is 40% or more,) (Table 5 (7)), similar results were obtained even when different protein sources were used in Test 3.

In addition, also in the rat (Table 9 (15)) that had ingested the enteral nutrient preparation (Example 6) in which soybean peptide was blended as protein source in refeeding, the plasma inorganic phosphorus concentration was significantly higher than that in Table 9 (13), and hypophosphatemia was suppressed.

In Test 1, it was suggested that by administering the enteral nutrient preparation containing the protein having the amino acid score of less than 100 as protein source to a subject in poor nutrition state, the hypophosphatemia was prevented or suppressed regardless of the lipid calorie ratio, but a similar result could be confirmed in Test 3.

For the test 3, Fisher's least significant difference method was used. The "n.s." and "$p < 0.05$" in Table 9 indicate that "there is no significant difference" and "there is a significant difference with $p < 0.05$", respectively as compared with the rat group that was taken low protein feed before refeeding and was refed with the enteral nutrient preparation of Comparative Example 6 (n = 8 (However, n = 7 in the group receiving the low protein feed before refeeding and receiving the enteral nutrient preparation of Comparative Example 6 in refeeding)).

[Table 10]

| Amino acid | Comparative example 1, 2, 4, 6 | Comparative example 3, example 3 | Example 1, 2 | Example 4, 6 | Comparative example 5, example 5 |
|---|---|---|---|---|---|
| Isoleucine | 5.7 | 3.1 | 1.3 | 3.9 | 3.5 |
| Leucine | 9.8 | 6.3 | 3.1 | 6.7 | 6.5 |
| Lysine | 12.9 | 7.4 | 8.0 | 10.7 | 9.5 |
| Methionine | 2.1 | 2.5 | 0.9 | 1.1 | 2.2 |
| Cysteine | 1.8 | 0.0 | 0.0 | 1.1 | 0.4 |
| phenylalanine | 2.5 | 4.0 | 2.1 | 4.5 | 3.5 |
| Tyrosine | 2.3 | 0.2 | 0.3 | 3.4 | 1.3 |
| Threonine | 6.6 | 2.6 | 2.0 | 3.7 | 2.8 |
| Tryptophan | 1.1 | 0.7 | 0.0 | 1.0 | 0.7 |
| Valine | 5.3 | 4.2 | 2.8 | 4.3 | 4.5 |
| Histidine | 1.6 | 1.7 | 0.7 | 2.5 | 1.8 |
| Arginine | 2.2 | 7.2 | 8.5 | 7.5 | 7.3 |
| Alanine | 5.5 | 8.4 | 9.9 | 3.8 | 6.9 |
| aspartic acid (asparagine + aspartic acid) | 10.5 | 5.0 | 5.9 | 11.6 | 7.0 |
| glutamic acid (glutamine + glutamic acid) | 18.1 | 9.2 | 10.8 | 19.8 | 12.5 |
| Glycine | 1.5 | 22.2 | 26.1 | 3.9 | 16.3 |
| Proline | 5.8 | 12.3 | 14.4 | 5.2 | 9.9 |
| Serine | 4.7 | 2.8 | 3.3 | 5.0 | 3.5 |
| Total | 100 | 100 | 100 | 100 | 100 |
| Amino acid score | 100 | 100 | 1 | 93 | 100 |

Claims

1.   An enteral nutrient preparation comprising protein and a lipid, and an amino acid as necessary, wherein 90 wt% or more of the protein is not milk-derived protein, and

(a) an amino acid score of a mixture of the protein and the amino acid is 100, and a lipid calorie ratio is 40% or more, or

(b) an amino acid score of a mixture of the protein and the amino acid is less than 100, and a lipid calorie ratio is 15% or more.

2. The enteral nutrient preparation according to claim 1, wherein 90 wt% or more of protein source of the protein is plant-derived protein and/or collagen.

3. The enteral nutrient preparation according to claim 1 or 2, comprising more than 3.5 g of the protein and/or the amino acid per 100 kcal in total.

4. The enteral nutrient preparation according to any one of claims 1 to 3, further comprising glucide.

5. The enteral nutrient preparation according to any one of claims 1 to 4, which is used for preventing or suppressing refeeding syndrome.

6. The enteral nutrient preparation according to any one of claims 1 to 5, which is used for preventing or suppressing hypophosphatemia.

7. The enteral nutrient preparation according to any one of claims 1 to 6, which is used for administration to a subject in poor nutrition state.

8. A method for providing nutritional supplementation to a subject in poor nutrition state using an enteral nutrient preparation, the enteral nutrient preparation comprising protein and a lipid, and an amino acid as necessary, wherein 90 wt% or more of the protein is not milk-derived protein, and

   (a) an amino acid score of a mixture of the protein and the amino acid is 100, and a lipid calorie ratio is 40% or more, or
   (b) an amino acid score of a mixture of the protein and the amino acid is less than 100, and a lipid calorie ratio is 15% or more.

Fig. 1

Plasma inorganic phosphorus concentrations (mg/dL)

Fig. 2

Plasma inorganic phosphorus concentrations (mg/dL)

Fig. 3

Plasma inorganic phosphorus concentrations (mg/dL)

Fig. 4

Plasma inorganic phosphorus concentrations (mg/dL)

| INTERNATIONAL SEARCH REPORT | International application No. |
|---|---|
| | **PCT/JP2022/026920** |

**A. CLASSIFICATION OF SUBJECT MATTER**

*A23L 33/17*(2016.01)i; *A23L 33/115*(2016.01)i; *A23L 33/125*(2016.01)i; *A61K 31/198*(2006.01)i; *A61K 31/20*(2006.01)i; *A61K 31/23*(2006.01)i; *A61K 36/48*(2006.01)i; *A61K 38/02*(2006.01)i; *A61K 38/39*(2006.01)i; *A61P 3/02*(2006.01)i; *A61P 3/12*(2006.01)i

FI: A23L33/17; A23L33/115; A23L33/125; A61K31/198; A61K31/20; A61K31/23; A61K36/48; A61K38/02; A61K38/39; A61P3/02; A61P3/12

According to International Patent Classification (IPC) or to both national classification and IPC

**B. FIELDS SEARCHED**

Minimum documentation searched (classification system followed by classification symbols)

A23L33/17; A23L33/115; A23L33/125; A61K31/198; A61K31/20; A61K31/23; A61K36/48; A61K38/02; A61K38/39; A61P3/02; A61P3/12

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2014-3960 A (NOF CORP.) 16 January 2014 (2014-01-16) claims, paragraphs [0024]-[0052], examples, tables 1, 17 | 1-4, 7, 8 |
| Y | | 1-8 |
| Y | 中屋豊他. "リフィーディング症候群". 四国医学雑誌. vol. 68, 2012, pp. 23-28, Internet: <URL: https://repo.lib.tokushima.ne.jp/ja/list/t-pubs/sam/68/1-2/item/97914>, (NAKAYA, Yutaka. Refeeding syndrome. Shikoku Acta Medica.) p. 24, left column, p. 26, left column, fig. 1, 2 | 1-8 |
| Y | 下村吉治. "分岐鎖アミノ酸（BCAA）：代謝特性と生理機能". 食品分析開発センターSUNATEC Webページ. 01 March 2021 [online], Internet: <URL: https://web.archive.org/web/20210301012417/http://www.mac.or.jp/mail/210301/02.shtml>, (Food Analysis Technology Center: Sunatec), non-official translation (SHIMOMURA, Yoshiharu. Branched-Chain Amino Acids (BCAA): Metabolic Characteristics and Physiological Functions.) 2., 3., table 1, fig. 2, 3 | 1-8 |

☐ Further documents are listed in the continuation of Box C.  ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **01 August 2022** | **23 August 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/026920**

| Patent document cited in search report | | | Publication date (day/month/year) | Patent family member(s) | | | Publication date (day/month/year) |
|---|---|---|---|---|---|---|---|
| JP | 2014-3960 | A | 16 January 2014 | CN | 103504293 | A | |
| | | | | claims, paragraphs [0039]-[0093], examples, tables 1, 17 | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HISHAM MEHANNA et al.** Refeeding syndrome - awareness, prevention and management. *Head & Neck Oncology,* 2009, vol. 1 (4

- **STANGA Z et al.** Nutrition in clinical practice - the refeeding syndrome: illustrative cases and guidelines for prevention and treatment. *Eur J Clin Nutr.,* 2008, vol. 62 (6), 687-94